(19) **Europäisches Patentamt**

**European Patent Office**

**Office européen des brevets**

(11) **EP 0 995 994 B1**

(12) **EUROPÄISCHE PATENTSCHRIFT**

(45) Veröffentlichungstag und Bekanntmachung des Hinweises auf die Patenterteilung:
**18.12.2002   Patentblatt 2002/51**

(51) Int Cl.7: **G01N 33/545**, G01N 33/52

(21) Anmeldenummer: **99120663.2**

(22) Anmeldetag: **19.10.1999**

(54) **Funktionsschichten mit hoher Präzision, Verfahren zu ihrer Herstellung und Teststreifen enthaltend diese Funktionsschichten**

High precision function layers, method for their preparation and test strips containing these function layers

Couches fonctionnelles à haute précision, méthode pour leur préparation et bandes d'essai contenants ces couches fonctionelles

(84) Benannte Vertragsstaaten:
**AT BE CH CY DE DK ES FI FR GB GR IE IT LI LU MC NL PT SE**

(30) Priorität: **23.10.1998   DE 19849000**

(43) Veröffentlichungstag der Anmeldung:
**26.04.2000   Patentblatt 2000/17**

(73) Patentinhaber: **Roche Diagnostics GmbH**
**68298 Mannheim (DE)**

(72) Erfinder:
• **Knappe, Wolfgang, Dr.**
  **67061 Ludwigshafen (DE)**
• **Mosoiu, Dan**
  **67117 Limburgerhof (DE)**

(56) Entgegenhaltungen:
**EP-A- 0 016 387**       **EP-A- 0 582 503**
**EP-A- 0 653 637**       **EP-A- 0 690 306**
**WO-A-92/15879**        **WO-A-97/18036**
**DE-A- 2 409 068**       **DE-A- 2 752 352**
**FR-A- 2 730 931**       **US-A- 4 370 412**
**US-A- 5 695 947**

**Beschreibung**

Die vorliegende Erfindung betrifft Funktionsschichten hoher Präzision und deren Herstellung, die Verwendung von N-Acyl-N-alkyl-glycinaten, N-Acyl-tauraten und/oder N-Acyl-glutamaten zur Herstellung dieser Funktionsschichten sowie Teststreifen, insbesondere diagnostische Teststreifen, die in ihren Testelementen mindestens eine erfindungsgemäße Funktionsschicht hoher Präzision tragen.

Zur qualitativen oder quantitativen analytischen Bestimmung von Bestandteilen von Körperflüssigkeiten. insbesondere von Blut, werden oft sogenannte trägergebundene Tests verwendet. Bei diesen liegen Reagenzien auf oder in entsprechenden Schichten eines festen Testträgers vor, der mit der Probe in Kontakt gebracht wird. Die Reaktion von flüssiger Probe und Reagenzien führt zu einem nachweisbaren Signal, insbesondere einer Farbänderung, welche visuell oder mit Hilfe eines Geräts, meistens reflektionsphotometrisch, ausgewertet werden kann.

Testträger sind häufig als Teststreifen ausgebildet, die im wesentlichen aus einer länglichen Tragschicht aus Kunststoffmaterial und darauf angebrachten Nachweisschichten als Testfelder bestehen. Es sind jedoch auch Testträger bekannt, die als quadratische oder rechteckige Plättchen gestaltet sind. In der folgenden Beschreibung soll die Bezeichnung "Teststreifen" auch Testträger umfassen, die nicht die Streifenform aufweisen.

Testträger der eingangs bezeichneten Art sind beispielsweise aus der deutschen Patentschrift 21 18 455 bekannt. Dort werden diagnostische Testträger zum Nachweis von Analyten in Flüssigkeiten beschrieben, die aus einer Tragschicht und mindestens einer die Nachweisreagenzien enthaltenden Nachweisschicht, deren nicht auf der Tragschicht anliegende Oberfläche mit einer Deckschicht versehen ist, bestehen. Die Deckschicht kann aus einem feinmaschigen Netzwerk in Form eines Gewebes, Gewirkes oder Vlieses bestehen. Kunststoffgewebe werden als bevorzugte Netzwerke angegeben, um eine schnelle Benetzung der Nachweisschicht mit Probenflüssigkeit zu erreichen und störende Chromatographieeffekte zu vermeiden. Zum Nachweis eines Analyts in einer Flüssigkeit wird ein solcher diagnostischer Testträger in eine entsprechende Flüssigkeit, vorzugsweise Urin, eingetaucht. Die Nachweisschicht kommt so mit einem sehr großen Überschuß an Flüssigkeit in Kontakt, der nicht von dem Testträger aufgenommen werden kann. Je nach der Dauer des Kontaktes der Nachweisschicht mit der zu untersuchenden Flüssigkeit können jedoch unterschiedliche Farbintensitäten beobachtet werden.

In der Regel werden um so positivere Resultate erhalten, je länger die Kontaktzeit ist. Bei einem großen Probenüberschuß ist daher eine korrekte quantitative Analytbestimmung auf diese Weise nicht möglich.

Andererseits ist ein für eine Testträgerkonstruktion zu geringes Probenvolumen eine häufige Ursache für falsche Meßwerte beim Diabetes-Monitoring, das heißt, der regelmäßigen Kontrolle des Blutes Diabeteskranker auf den Gehalt an Glucose.

Testträger mit möglichst geringem Volumenbedarf sind deshalb das Ziel vielfältiger derzeitiger Entwicklungen. Solche Testträger müssen jedoch nicht nur mit sehr kleinen Probenvolumina von etwa 3μl richtige Meßwerte ergeben, sondern sie müssen auch bei relativ großen Probenvolumina von etwa 15-20 μl zuverlässig arbeiten und müssen die Probenflüssigkeit halten. Im Falle eines Herauslaufens von Flüssigkeit aus dem Testträger können nämlich hygienische Probleme auftreten, beispielsweise dann, wenn potentiell infektiöses Fremdblut vermessen wird oder wenn der Testträger apparativ vermessen werden soll und dann die Gefahr der Verschmutzung des Meßgerätes besteht.

Aus der DE-A-3042857 sind Teststreifen bekannt, die auf ihren Analyseelementen eine Probenverteilungsschicht (Spreitschicht) aufweisen, die die Aufgabe hat, punktförmig aufgetragene Probeflüssigkeit gleichmäßig über das ganze Testelement zu verteilen. Diese Spreitschicht besteht aus einem Tuch oder einer Schaumstoffschicht, die durch Imprägnierung mit einem Netzmittel hydrophiliert und entweder auf die noch feuchte oberste Gelatineschicht des Analyseelements gepreßt oder mittels einer zusätzlichen Klebstoffschicht darauf fixiert wird.

Die Analyseelemente dieser bekannten Teststreifen, die in der Literatur und in der folgenden Beschreibung synonym als Testelemente, Testfelder, Nachweiselemente, Nachweisfelder oder als Nachweisschichten bezeichnet werden, weisen zwei oder mehr Schichten auf, die die zum Nachweis und der quantitativen Bestimmung des Analyts (hier Harnstoff) erforderlichen Reagenzien oder Hilfsstoffe, wie z.B. strahlungsabsorbierende Stoffe, enthalten. Im Folgenden werden derartige Schichten als Funktionsschichten bezeichnet.

Diagnostische Testträger in Form von Teststreifen, die einen deutlichen Fortschritt in Bezug auf Reproduzierbarkeit der Testergebnisse selbst bei Applikation unterschiedlicher Probenvolumina und in Bezug auf hygienische Handhabbarkeit bieten, sind aus der EP-A-0 821 233 bekannt.

Sie enthalten eine Tragschicht mit einer darauf angeordneten Nachweisschicht, die eine oder mehrere Funktionsschichten aufweist, welche die zur Bestimmung von Analyt in flüssiger Probe erforderliche Reagenzien enthalten, und eine die Nachweisschicht überdeckende Auflage aus einem hydrophilen, aber nicht kapillaraktiven, relativ grobmaschigen Netzwerk, die größer als die Nachweisschicht ist und die beiderseits der Nachweisschicht auf der Tragschicht befestigt ist, auf der Nachweisschicht dagegen ohne Befestigung unmittelbar, d.h. diese ohne Abstand im wesentlichen vollflächig berührend, aufliegt.

Dieses Netzwerk leitet auf seine Oberfläche aufgebrachte Probenflüssigkeit schnell auf die darunterliegende Nachweisschicht weiter und führt unter Mitwirkung einer die Randbereiche des Netzwerks abdeckenden Folienschicht eventuell vorhandene Probenüberschüsse in die über die Nachweisschicht hinausgehenden Randbereiche des Netzwerks ab. Auf diese Weise werden geringe Probenmengen der Nachweisschicht vollständig zur Verfügung gestellt, Fehl-Positiv-Ergebnisse aber vermieden.

Das Bemühen im Stand der Technik geht offensichtlich dahin, unter Verwendung von Teststreifen mit immer geringeren Probenvolumina möglichst genaue quantitative Bestimmungen der interessierenden Analyte zu erzielen. Hand in Hand mit der Verbesserung der Konstruktion der Teststreifen geht auch eine Reduzierung der für die Analyse verwendeten Nachweisflächen. So wird beispielsweise bei einem bekannten, zur Routinebestimmung von Blutzucker häufig eingesetzten Gerät, dem GLUCOTREND®-Gerät, nur noch eine Fläche von ca. 1 mm Durchmesser der Nachweisschicht ausgewertet.

Dieser Trend hat den Vorteil, daß die kleinen Probenmengen auf den kleinen Flächen noch gut erkennbare Farbsignale hervorbringen, er birgt jedoch das Risiko, das bereits geringfügige örtliche Unterschiede in der Struktur der Funktions- bzw. Nachweisschichten zu gravierenden Fehlmessungen führen können.

Herkömmliche Funktionsschichten können ein Skelett aus einem faserigen oder nichtfaserigen porösen Werkstoff enthalten, in den die für den Nachweis der Analyte erforderlichen Reagenzien und Hilfs- und Zusatzstoffe eingearbeitet sind.

Grundsätzlich ist es erforderlich für Funktionsschichten solche Materialien einzusetzen, die in der Lage sind, die zu untersuchende Flüssigkeit mit darin enthaltenen Inhaltsstoffen aufzunehmen. Dies sind sogenannte saugfähige Materialien, wie beispielsweise Vliese, Gewebe, Gewirke, Membranen oder sonstige poröse Kunststoffmaterialien, die als Skelett für die Schicht verwendet werden können und die natürlich die Struktur und Dimensionen der Schicht maßgeblich bestimmen. Die für die Nachweisschicht in Frage kommenden Materialien müssen natürlich auch Reagenzien tragen können, die für den Nachweis des zu bestimmenden Analyts erforderlich sind. Im einfachsten Fall befinden sich alle für den Nachweis des Analyts erforderliche Reagenzien auf oder in einem solchen Skelettmaterial.

Häufig eingesetzte Skelettmaterialien für die Funktionsschicht sind Papiere, die oben genannten textilen Flächengebilde aus natürlichen oder synthetischen Fasern oder poröse Kunststoffmaterialien, wie Membranen insbesondere asymmetrisch poröse Membranen, wobei in der Regel die zu untersuchende Probenflüssigkeit auf die großporige Seite der Membran aufgegeben wird und die Bestimmung des Analyts von der feinporigen Seite der Membran aus erfolgt. Als poröse Membranmaterialien sind Polyamid-, Polyvinylidendifluorid-, Polyethersulfon- oder Polysulfonmembranen, insbesondere Polyamid 66-Membranen und hydrophilisierte asymmetrische Polysulfonmembranen eingesetzt worden. Die Reagenzien zur Bestimmung des nachzuweisenden Analyts sind in der Regel durch Imprägnierung in die vorstehend genannten Materialien eingebracht oder durch Beschichtung einseitig aufgebracht worden. Bei Beschichtung asymmetrischer Membranen wird vorteilhafterweise die feinporige Seite beschichtet.

Naturgemäß bringen die Skelettmaterialien eine Inhomogenität in die Schichten hinein, die sich bei großflächiger Auswertung der durch den Analyt erhaltenen Farbsignale herausmitteln, bei zunehmend kleineren ausgewerteten Flächen aber störend bemerkbar machen können.

Es sind daher verschiedene Ansätze gemacht worden, skelettfreie Funktionsschichten in hoher Präzision, d. h. mit möglichst wenigen und geringen örtlichen Inhomogenitäten zu erzeugen.

Dabei ist zu beachten, daß die Schichten auf den Unterlagen gut verlaufen und haften. Das Bemühen geht dahin, die Oberflächenspannung der Schichtmaterialien herabzusetzen bzw. aneinander anzunähern. Hierzu wurde der Zusatz von organischen Lösungsmitteln, wie z.B. 1-Hexanol, oder von Netzmitteln vorgeschlagen. Ein anderer Ansatz setzt auf eine Oberflächenbehandlung der jeweiligen Unterlage, auf die die Schicht aufgebracht werden soll, durch eine Plasmaentladung (Corona-Behandlung). Auch die Kombination dieser Maßnahmen ist möglich.

Diese Maßnahmen des Standes der Technik weisen jedoch erhebliche Nachteile auf. Organische Lösungsmittel wie z.B. das 1-Hexanol können nicht in größeren Konzentrationen eingesetzt werden, da sie in Wasser, der bevorzugten flüssigen Phase von Beschichtungsmassen, schlecht löslich sind. Wassermischbare Lösungsmittel, wie z.B. Aceton, senken die Oberflächenspannung der Beschichtungsmassen ebenfalls, können jedoch nur sehr begrenzt eingesetzt werden, da sie die als Unterlage für die erste Schicht eingesetzten Kunststoffolien anlösen, wodurch diese sich krümmen und auch die erhaltenen Funktionsschichten gekrümmt sind. Derartige Schichten sind nicht einwandfrei zu verarbeiten und zu vermessen.

Netzmittel sind nicht generell einsetzbar, insbesondere sind sie bei der Herstellung von Glucosenachweisschichten limitiert, da sie weder das verwendete Enzym Glucose-dye-oxidoreductase (Gluc-DOR) denaturieren noch die Erythrozyten der aufgetragenen Blutprobe lysieren dürfen, weil sonst durch die Rotfärbung der Meßseite der Nachweisschicht des Teststreifens die Erkennbarkeit und Meßbarkeit des Farbsignals der Nachweisreaktion erheblich beeinträchtigt wird.

Coronabehandlungen der Unterlagen stellen in erster Linie einen zusätzlichen Arbeitsschritt dar und erfordern darüberhinaus eine entsprechende kostspielige Apparatur, die einen störanfälligen Teil der Produktionslinie darstellt.

Aus DE-A 24 09 068 sind skelettfreie Funktionsschichten für Rasterbilder bekannt. Sie enthalten Inertgelatine als Filmbildner, in die u.a. Hydrochinon und Kaliumchromalaun als funktionelle Verbindungen eingelagert sind. Darüberhinaus enthält die Funktionsschicht ein wasserlösliches Netzmittel, u.a. das Natriumsalz des Ölsäuremethyltaurins. Ähnliche Funktionsschichten, die ebenfalls in der Photographie Verwendung finden, sind aus US 4,370,412 bekannt. Sie enthalten ein Gemisch aus zwei Netzmitteln, u.a. Sarcosinatderivate in Mengen von 0,01 bis 2 Gew.-%. Netzmittelgemische sind weiterhin in EP-A 0 582 503 und FR-A 2 730 931 beschrieben, jedoch nicht im Zusammenhang mit Funktionsschichten.

Teststreifen, die skelettfreie Funktionsschichten enthalten, deren Herstellung und Verwendung sind beispielsweise aus EP-A 0 016 387, EP-A 0 653 637 und WO-A 92/15879 bekannt.

Es besteht daher nach wie vor ein dringendes Bedürfnis, skelettfreie Funktionsschichten mit hoher Präzision herstellen zu können, d.h. solche, die keine oder möglichst wenige und geringe örtliche Unregelmäßigkeiten aufweisen.

Die vorliegende Erfindung kommt diesem Bedürfnis entgegen.

Gegenstand der Erfindung sind Funktionsschichten gemäß Anspruch 1, Verfahren zu deren Herstellung gemäß Anspruch 10, Teststreifen enthaltend erfindungsgemäße Funktionsschichten gemäß Anspruch 13, sowie deren Verwendung gemäß Anspruch 17 und entsprechende Bestimmungsverfahren gemäß Anspruch 18.

Ein Gegenstand dieser Erfindung ist eine skelettfreie Funktionsschicht mit hoher Präzision umfassend einen Film aus einem natürlichen oder synthetischen filmbildenden Polymer (Filmbildner), einer oder mehreren, die Funktion der Schicht ermöglichenden Verbindungen, gegebenenfalls Hilfs- und/oder Zusatzstoffe, die dadurch gekennzeichnet ist, dass die Schicht Netzmittel der Formel I, II und/oder III enthält,

$$R^1\text{-CO-N}(R^2)\text{-CH}_2\text{-COOMe} \tag{I}$$

$$R^1\text{-CO-N}(R^2)\text{-CH}_2\text{-CH}_2\text{-SO}_3\text{Me} \tag{II}$$

$$HO_2C\text{-CH}_2\text{-CH}_2\text{-CH(NH-COR}^1)\text{-CO}_2\text{Me} \tag{III}$$

worin $R^1$ ein vorzugsweise geradkettiger oder ein wenig verzweigter aliphatischer Rest mit 9 bis 23 C-Atomen, insbesondere mit 11 bis 19 C-Atomen ist, der gesättigt ist oder eine bis drei Doppelbindungen aufweist,

$R^2$ ein vorzugsweise geradkettiger oder wenig verzweigter Alkylrest mit 1 bis 8, vorzugsweise 1 bis 4, C-Atomen ist, und Me für Wasserstoff- oder Metallatom steht.

Vorzugsweise ist $R^1$ die aliphatische Kette der Laurinsäure, der Myristinsäure, der Palmitinsäure, der Stearinsäure der Palmitoleinsäure, der Ölsäure, der Linolsäure, der Linolensäure oder deren Isomeren und $R^2$ Methyl oder Ethyl.

Von besonderer ökonomischer Bedeutung und sehr guter Wirksamkeit sind auch Verbindungen der Formeln I, II und III in denen $R^1$ für eine Menge von Alkylresten, in der die einzelnen Alkylreste bezüglich ihrer Struktur und ihres Anteils in der Mischung der Struktur und dem Anteil ihres Vorkommens in natürlichen Fetten entsprechen, steht.

Besonders bevorzugt sind beispielsweise Verbindungen der Formeln I, II und III in denen $R^1$-CO- ein Oleoyl-, Kokoyl- oder Talgfettsäurerest und $R^2$ Methyl ist.

Zweckmäßigerweise wird das für Me stehende Metallatom so gewählt, daß die Verbindungen der Formel I, II, und III wasserlöslich sind. Vorzugsweise bedeutet Me ein Alkalimetallatom insbesondere ein Natrium- oder Kaliumatom.

Erfindungsgemäße skelettfreie Funktionsschichten können als Netzmittel eine Mischung von Verbindungen der Formel I, II und/oder III ist.

Aus wirtschaftlichen Gründen besonders günstig sind erfindungsgemäße skelettfreie Funktionsschichten, die Handelsprodukte der Formeln I oder II enthalten. Gut geeignete Handesprodukte sind beispielsweise Natrium-N-oleoyl-sarcosinat, leicht erhältlich aus N-Oleoyl-sarcosin (z.B. ®Crodasinic O der Fa. Croda, Nettetal) und NaOH und Natrium-N-methyl-N-oleoyl-taurat (z.B. ®Geropon T 77 der Fa. Rhone-Poulenc Chimie, Paris) und Mononatrium-N-cocoyl-L-glutamat (z.B. ®Aminosoft CS-11 der Fa. Ajinomoto, Tokio).

Sehr überraschend ist, daß die hohe Präzision der erfindungsgemäßen Funktionsschichten bereits erreicht wird, wenn sie insgesamt 0,0075 bis 2,5 Gew.-%, vorzugsweise 0,01 bis 2,0 Gew.-%, insbesondere 0,03 bis 1,0 Gew.-%, Netzmittel der Formel I, II und/oder III enthalten.

Die hohe Präzision der erfindungsgemäßen Funktionsschichten äußert sich, wenn diese als Nachweisschichten in Teststreifen eingebracht werden, in einer erheblichen Zunahme der Genauigkeit qualitativer und quantitativer Bestimmungen von Analyten. Die Verbindungen der Formeln I, II und/oder III enthaltenden, erfindungsgemäßen Beschichtungsmassen zeigen auch eine erheblich niedrigere Viskosität als Beschichtungmassen gleicher Zusammensetzung aber ohne Verbindungen I, II oder III, wobei in vielen Fällen das Ausmaß der Viskositätsabnahme um so größer ist, je höher die Viskosität der Masse vor dem Zusatz der Verbindungen I, II oder III ist. Dies führt dazu, daß die Viskositätsunterschiede von verschieden zusammengesetzten Beschichtungsmassen kleiner werden, sodaß sich viskositätsabhängige Verarbeitungseigenschaften verschiedener Massen aneinander angleichen, was einen großen Vorteil beispielsweise beim maschinellen Gießen der Schichten bringt.

Visuell wird beobachtet, daß erfindungsgemäße Beschichtungsmassen kurz nach dem Aufbringen und vor dem Trocknen eine spiegelglatte Oberfläche zeigen. Sie verlaufen also sehr gut auf der Trägerfolie bzw. auf der darauf bereits vorhandenen ersten (trockenen) Schicht. Bei Schichten ohne Zusatz der Verbindungen I, II oder III erscheint die Oberfläche visuell deutlich unebener.

Die erfindungsgemäßen Funktionsschichten dienen vorzugsweise als Nachweisschichten oder sind Bestandteil von Nachweisschichten in Teststreifen, insbesondere von diagnostischen Teststreifen und enthalten als solche neben den Filmbildnern Reagenzien oder Hilfs- und/oder Zusatzstoffe zum qualitativen Nachweis oder zur quantitativen Bestimmung von Analyten.

Im Einzelnen ist zu der Zusammensetzung der erfindungsgemäßen Funktionsschichten Folgendes zu sagen:

Die Funktionsschichten werden aus Dispersionen oder Emulsionen polymerer Filmbildner hergestellt Dispersionsfilmbildner enthalten mikroskopische, in der Trägerflüssigkeit (meist Wasser) unlösliche Polymerteilchen, welche in feinster Verteilung in der Trägerflüssigkeit dispergiert sind. Wird bei der Filmbildung die Flüssigkeit durch Verdampfen entfernt, so nähern sich die Teilchen und berühren sich schließlich. Durch die dabei auftretenden großen Kräfte und einen mit der Filmbildung einhergehenden Abbau der Oberflächenenergie wachsen die Teilchen zu einer weitgehend geschlossenen Filmschicht zusammen. Alternativ kann auch eine Emulsion des Filmbildners verwendet werden, bei der dieser in einem Lösungsmittel gelöst ist. Das gelöste Polymer ist in einer Trägerflüssigkeit emulgiert, die mit dem Lösungsmittel nicht mischbar ist.

Als Polymere für solche Filmbildner eignen sich insbesondere Polyvinylester, Polyvinylacetate, Polyacrylester, Polymethacrylsäure, Polyvinylamide, Polyamide und Polystyrol. Neben Homopolymeren sind auch Mischpolymerisate, z. B. von Butadien, Styrol oder Maleinsäureester geeignet.

Wird eine erfindungsgemäße Funktionsschicht mit einer weiteren erfindungsgemäßen oder auch einer nicht erfindungsgemäßen Funktionsschicht zu einer Nachweisschicht kombiniert, so können die Funktionsschichten aus Beschichtungsmassen hergestellt werden, die den gleichen polymeren Filmbildner enthalten oder sie können aus Beschichtungsmassen erzeugt werden, die unterschiedliche polymere Filmbildner enthalten.

Für die Funktionsschicht kommen jedoch auch sogenannte offene Filme in Frage, wie sie beispielsweise in EP-B-0 016 387 beschrieben sind. Hierfür werden einer wäßrigen Dispersion von filmbildenden organischen Kunststoffen Feststoffe als feine, unlösliche, organische oder anorganische Partikel zugegeben und die für die Nachweisreaktion erforderlichen Reagenzien zusätzlich hinzugefügt. Geeignete Filmbildner sind bevorzugt organische Kunststoffe, wie Polyvinylester, Polyvinylacetate, Polyacrylester, Polymethacrylsäure, Polyacrylamide, Polyamide, Polystyrol, Mischpolymerisate, zum Beispiel von Butadien und Styrol oder von Maleinsäureester und Vinylacetat oder andere filmbildende, natürliche und synthetische organische Polymere sowie Mischungen derselben in Form von wäßrigen Dispersionen. Die Dispersionen lassen sich auf einer Unterlage zu einer gleichmäßigen Schicht verstreichen, die nach dem Trocknen einen wasserfesten Film ergibt. Die trocknen Filme haben eine Dicke von 10 µm bis 500 µm, vorzugsweise von 30 bis 200 µm. Der Film kann mit der Unterlage als Träger zusammen verwendet werden oder für die Nachweisreaktion auf einen anderen Träger aufgebracht werden. Obwohl die für die Nachweisreaktion erforderlichen Reagenzien normalerweise in die zur Herstellung der offenen Filme verwendete Dispersion gegeben werden, kann es auch vorteilhaft sein, wenn der gebildete Film nach seiner Herstellung mit den Reagenzien imprägniert wird. Auch eine Vorimprägnierung der Füllstoffe mit den Reagenzien ist möglich.

Ein weiteres Beispiel für eine erfindungsgemäß bevorzugte Nachweisschicht ist eine Filmschicht, wie sie in WO-A-92 15 879 beschrieben ist. Diese Schicht wird aus einer Dispersion oder Emulsion eines polymeren Filmbildners hergestellt, welche zusätzlich in homogener Verteilung ein Pigment, ein Quellmittel und das Nachweisreagenz enthält. Als polymere Filmbildner eignen sich insbesondere Polyvinylester, Polyvinylacetate, Polyacrylester, Polymethacrylsäure, Polyvinylamide, Polyamide und Polystyrol. Neben Homopolymeren sind auch Mischpolymerisate, z.B. von Butadien, Styrol oder Maleinsäureester geeignet. Titandioxid ist ein für den Film besonders geeignetes Pigment. Das verwendete Quellmittel soll besonders gute Quelleigenschaften aufweisen, wobei Methylvinylethermaleinsäure-Copolymer besonders empfohlen wird.

Neben den Filmbildnern und den Reagenzien für den Analyt-Nachweis kommt Hilfs- und/oder Zusatzstoffen eine große Bedeutung als Bestandteil der erfindungsgemäßen Funktionsschichten zu. Sie dienen dazu, die Schichten speziellen Anforderungen anzupassen beispielsweise die Erkennbarkeit der Farbreaktionen zu verbessern, die Reagenzsysteme zu stabilisieren und/oder die Probenflüssigkeit durch eine Filterwirkung "aufzubereiten", z.B. durch Abtrennung bestimmter den Nachweis störender Inhaltsstoffe. Beispiele für derartige Zusatzstoffe sind Pigmente mit ausgewählten Brechungsindices, Quellmittel oder nicht poröse oder poröse Füllstoffe wie Kieselgur (Diatomeenerde).

Durch Zugabe eines gut quellenden Quellmittels (das heißt, einer Substanz, die unter Aufnahme von Wasser ihr Volumen vergrößert) erhält man nicht nur Schichten, die relativ schnell von Probenflüssigkeit penetriert werden, sondern die trotz dieser Öffnungswirkung des Quellmittels gute Erythrozyten- und außerdem auch Blutfarbstoffabtrenneigenschaften besitzen. Die Quelleigenschaften sollten so gut sein, daß für einen Test, bei dem die Geschwindigkeit der Farbbildung - wie beispielsweise einer Glucosenachweisreaktion - überwiegend von der Penetration der Probenflüssigkeit durch die Schicht abhängt, die optisch nachweisbare Reaktion nach maximal einer Minute meßbar ist. Als besonders geeignete Quellmittel haben sich Xanthangum und Methylvinylethermaleinsäure-Copolymer erwiesen.

Reagenzsysteme zum Nachweis bestimmter Analyte durch Farbbildung sind dem Fachmann bekannt. Es ist möglich, daß sich sämtliche Komponenten des Reagenzsystems in einer Filmschicht befinden. Es ist aber auch möglich, daß die Komponenten des Reagenzsystems auf beide Filmschichten verteilt sind. Vorteilhafterweise befindet sich das farbbildende Reagenzsystem wenigstens zum Teil in der ersten Filmschicht.

Unter Farbbildung wird im Rahmen der vorliegenden Erfindung nicht nur der Übergang von weiß nach farbig verstanden, sondern auch jede Farbveränderung, wobei natürlich solche Farbveränderungen besonders bevorzugt sind, die mit einer möglichst großen Verschiebung der maximalen Absorptionswellenlinie ($\lambda$ max) einhergehen.

Die erfindungsgemäßen Schichten werden auf einer Unterlage erzeugt. Dies ist zweckmäßigerweise eine, vorzugsweise transparente, Folie als Basisunterlage oder eine bereits mit einer erfindungsgemäßen oder einer nicht erfindungsgemäßen Schicht versehene Folie. Als Basis-Unterlage kommen insbesondere solche Kunststoffolien in Betracht, die flüssigkeitsundurchlässig sind. Polycarbonatfolie hat sich als besonders bevorzugt erwiesen.

Die Dicke der erfindungsgemäßen Funktionsschichten beträgt in der Regel nicht mehr als 0,1 mm, vorzugsweise nicht mehr als 0,05 mm.

Neben den oben beschriebenen erfindungsgemäßen skelettfreien Funktionsschichten hoher Präzision ist auch die Verwendung von Verbindungen der Formel I und/oder II, worin die Reste $R^1$, $R^2$, $R^3$ und Me die in Anspruch 1 angegebenen Bedeutungen haben, zur Herstellung von skelettfreien Funktionsschichten hoher Präzision ein Gegenstand der vorliegenden Erfindung.

Ein weiterer Gegenstand der Erfindung ist ein Verfahren zur Herstellung von skelettfreien Funktionsschichten hoher Präzision durch Beschichen einer Unterlage mit einer flüssigen oder pastösen Beschichtungsmasse aus einem flüssigen Lösungsoder Dispergiermittel, einer Lösung, einer Dispersion oder einem redispergierbaren oder löslichen Präparat eines natürlichen oder synthetischen filmbildenden Polymers (Filmbildners), einer oder mehreren, die Funktion der Schicht ermöglichenden Verbindungen und gegebenenfalls Hilfs und/oder Zusatzstoffen, und anschließendes Entfernen des flüssigen Lösungs- oder Dispergiermittels, dadurch gekennzeichnet, daß die flüssige oder pastöse Mischung ein Netzmittel der Formel I, II und/oder III enthält, worin die Reste $R^1$, $R^2$ und Me die oben angegebenen Bedeutungen haben.

Lösungen im Sinne dieser Verfahrensbeschreibung sind auch sog. Scheinlösungen, d.h. Systeme aus Lösungsmittel und hochmolekularen Substanzen, die keine Phasengrenzen aufweisen aber einen Tyndall-Effekt zeigen. Unter den Begriff der Dispersion sollen alle Systeme aus einer kontinuierlichen flüssigen Phase, dem Dispergiermittel, und einer darin fein verteilten diskontinuierlichen Phase fallen, also auch Emulsionen. Geeignete Gruppen natürlicher und synthetischer Filmbildner sind bereits oben bei der stofflichen Beschreibung der erfindungsgemäßen Funktionsschichten ausführlich behandelt worden.

Die bei dem erfindungsgemäßen Verfahren eingesetzte flüssige oder pastöse Beschichtungsmasse enthält insgesamt 0,0075 bis 2,5 Gew.-%, vorzugsweise 0,01 bis 2,0 Gew.-%, insbesondere 0,03 bis 1,0 Gew.-%, bezogen auf das Gewicht aller Bestandteile der Beschichtungsmasse mit Ausnahme des Wassers, von Netzmitteln der Formel I, II und/oder III, worin die Reste $R^1$, $R^2$, und Me die oben angegebenen Bedeutungen haben.

Die Unterlage, auf der eine erfindungsgemäße Schicht erzeugt wird, kann bereits eine oder mehrere erfindungsgemäße oder nicht erfindungsgemäße Funktionsschichten tragen.

Die Beschichtung kann durch alle bekannten Beschichtungsverfahren ausgeführt werden, die einen dosierten Auftrag von Beschichtungsmasse ermöglichen, insbesondere durch Gießen, durch Aufstreichen mittel Bürsten, Pinsel oder Rakel oder durch Walzenauftrag oder durch Kombinationen dieser Verfahren. So kann eine durch Walzen aufgebrachte Schicht durch Bürsten oder gezielte Luftströme (sog. Luftbürsten) egalisiert werden; Rakeln verschiedener bekannter Konstruktionen können zur Entfernung eines eventuell aufgetragenen Überschusses an Beschichtungsmasse eingesetzt werden.

Die Entfernung der flüssigen Phase des Beschichtungsmittels erfolgt, sofern das Material der Unterlage und/oder die sonstigen Inhaltsstoffe der Beschichtungsmasse keine anderen Grenzen setzen, zweckmäßigerweise bei Temperaturen zwischen Raumtemperatur und dem Siedepunkt der flüssigen Phase, vorzugsweise bei Temperaturen von ca. 40 bis 80°C.

Ein weiterer Gegenstand der vorliegenden Erfindung sind Teststreifen aus einem - in der Regel flexiblen - flächenförmigen Träger, auf dem in einem Testbereich ein oder mehrere Testelemente nebeneinander angeordnet sind, die jeweils eine oder mehrere übereinanderliegende Funktionsschichten umfassen, und gegebenenfalls durch eine Auflage aus einem Spreitmaterial abgedeckt sind, die dadurch gekennzeichnet sind, daß mindestens eine der Funktionsschichten eine erfindungsgemäße skelettfreie Funktionsschicht hoher Präzision ist.

Die aus einer oder mehreren Funktionsschichten aufgebauten Testelemente können, wie oben bereits gesagt, auch als Testfelder, Nachweiselemente, Nachweisfelder oder als Nachweisschichten bezeichnet werden.

Auch in der folgenden Beschreibung werden diese Ausdrücke synonym benutzt.

Besonders vorteilhaft vom Standpunkt der Arbeitsökonomie sind solche erfindungsgemäßen Teststreifen, die zwei unmittelbar aneinandergrenzende oder durch einen Spalt getrennte ein- oder mehrschichtige Testfelder für den gleichen oder für verschiedene Analyte aufweisen.

Sollen, was bevorzugt ist, die Testreifen diagnostischen Zwecken dienen, so enthalten die Testfelder in ihren Funktionsschichten Reagenzien für den Nachweis eines diagnostisch verwertbaren Analyts.

Der flächenförmige Träger (Tragschicht) des erfindungsgemäßen Teststreifens besteht zweckmäßigerweise aus einem Material, das die zu untersuchende Flüssigkeit nicht aufnimmt. Dies sind sogenannte nicht-saugfähige Materialien, wobei Kunststoffolien beispielsweise aus Polystyrol, Polyvinylchlorid, Polyester, Polycarbonat oder Polyamid besonders bevorzugt sind. Es ist jedoch auch möglich, saugfähige Materialien, wie zum Beispiel Holz, Papier oder Pappe mit wasserabstoßenden Mitteln zu imprägnieren oder mit einem wasserfesten Film zu überziehen, wobei als Hydrophobierungsmittel Silikone oder Hartfette und als Filmbildner beispielsweise Nitrocellulose oder Celluloseacetat verwendet werden können. Als weitere Trägermaterialien eignen sich Metallfolien oder Glas.

Da in der Regel die visuelle Beurteilung und/oder die apparative Ausmessung des in der Regel in Form von Farbänderungen der Nachweisschicht erhaltenen Testergebnisses von der, der Probe-Aufgabeseite gegenüberliegenden Seite des Nachweiselements erfolgt, ist es erforderlich, daß der Träger des Teststreifens aus einem transparenten Material besteht und/oder im Bereich des Testfeldes eine Lochung aufweist, die von der Nachweisschicht überdeckt ist. Durch die Lochung ist dann die Nachweisschicht, zumindest aber die Reaktionsbezirke der Nachweisschicht sichtbar. Durch ein Loch können aber auch mehrere Reaktionsbezirke einer Nachweisschicht sichtbar sein.

Insbesondere bei bevorzugten Teststreifen mit zwei oder mehreren nebeneinander angeordneten Nachweiselementen kann die Lochung eines erfindungsgemäßen diagnostischen Testträgers auch aus zwei oder mehr Löchern gleicher oder unterschiedlicher Form bestehen, die zur Bestimmung von Analyt (einem oder mehreren Analyten) genutzt werden können. Über den Löchern können die verschiedenen Nachweisschichten angeordnet sein oder auch nur eine Nachweisschicht mit mehreren Reaktionsbezirken, so daß durch je 1 Loch eine Nachweisschicht oder je ein Reaktionsbezirk beobachtet werden kann.

In einer bevorzugten Ausführungsform des erfindungsgemäßen diagnostischen Testträgers befindet sich in der Tragschicht unterhalb einer Nachweisschicht ein Loch, durch das die Nachweisschicht oder ein Reaktionsbezirk beobachtbar ist, das einen etwas kleineren Durchmesser besitzt als die kleinste Längenausdehnung der Nachweisschicht, so daß die Nachweisschicht außerhalb des Loches auf der Tragschicht aufliegt und dort mittels dünnem Klebeband auf der Tragschicht befestigt sein kann. In der Regel ist die Nachweisschicht durch beidseitig daneben angeordnete doppelseitige Klebebänder und die über der Nachweisschicht liegende i Spreitauflage und deren Befestigung auf der Tragschicht ausreichend fixiert.

Um die Ausmessung der Testreaktion einfach und dennoch reproduzierbar zu gestalten, weist der Träger des Teststreifens zweckmäßigerweise Justierungsmarkierungen, z.B. Kerben oder Bohrungen auf, in entsprechende Justierungselemente des eingesetzten Meßgerätes - z.B. eines ®GLUCOTREND-Gerätes - eingreifen und so eine korrekte Lage des Teststreifens im Meßgerät sicherstellen.

Zur Veranschaulichung dieser und der folgenden Ausführungen dienen die Figuren 1 bis 4.

Die Figur 1 zeigt eine perspektivische Aufsicht, die Figur 2 einen Schnitt längs der Schnittlinie A-A', die Figur 3 eine Aufsicht auf die Unterseite einer Ausführungsform eines erfindungsgemäßen Teststreifens.

Die Figur 4 zeigt einen vergrößerten Ausschnitt aus dem in Figur 2 gezeigten Schnitt.

Die in den Figuren verwendeten Bezugszeichen haben folgende Bedeutung:

1:         Teststreifen

2:         Flexibler Träger

3 bis 3c:         Erfindungsgemäße Funktionsschichten

4 und 4a:         Abstandshalter

5 und 5a:         Adhäsionsschichten

6:         Spreitauflage

7 und 7a:         Schutzabdeckung

8:         Auftragsbereich für Probenmaterial

9:          Markierung der Einschubrichtung

10:         Positionierungsbohrung

11 und 12:   Beobachtungs- und Meßöffnungen

13:         Basis-Folienschicht

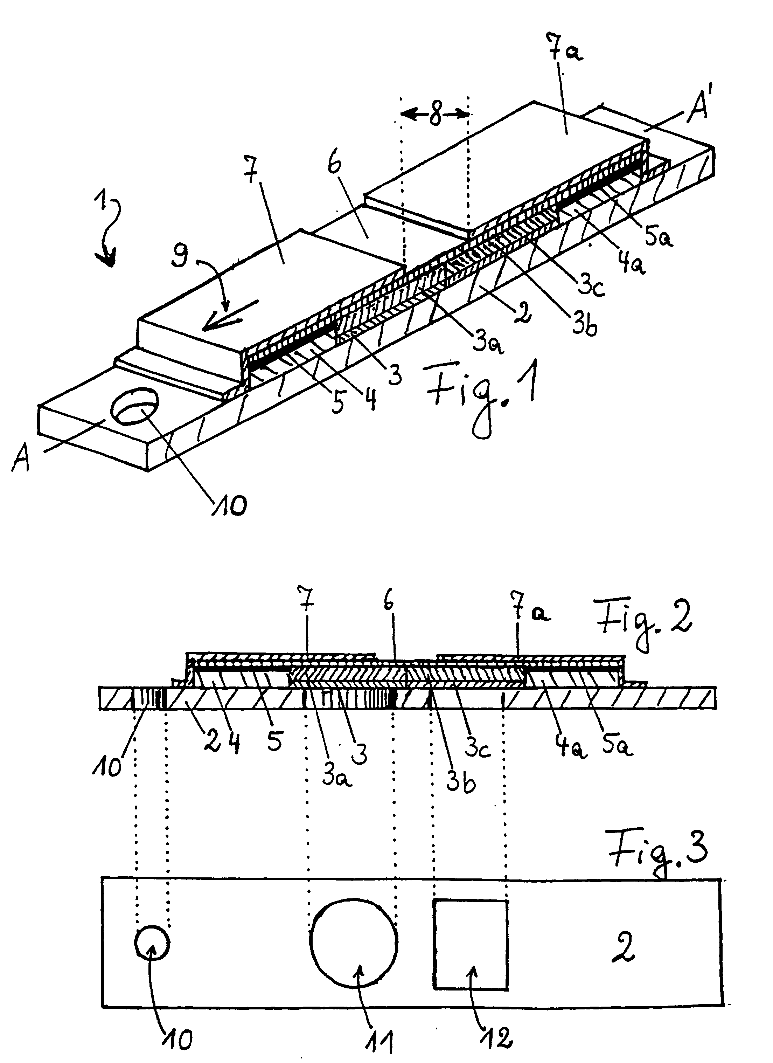

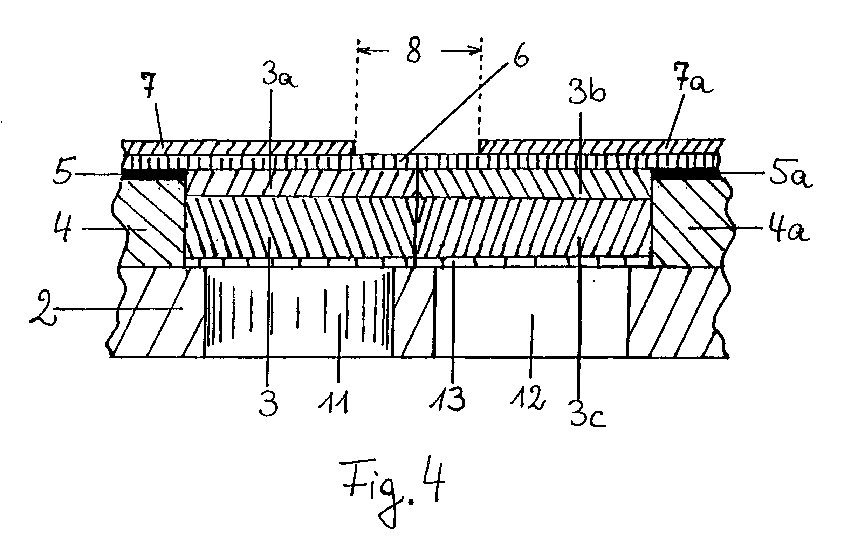

Die Figur 1 zeigt eine perspektivische Aufsicht, die Figur 2 einen Schnitt längs der Schnittlinie A-A'in Figur 1, die Figur 3 eine Aufsicht auf die Unterseite einer Ausführungsform eines erfindungsgemäßen Teststreifens mit einer Nachweisfläche und einer erfindungsgemäßen Spreitauflage. Diese Darstellung erfolgt ohne Maßstab um den Aufbau klar ersichtlich machen zu können. Eine konkrete Dimensionierung dieser Ausführungsform kann dem Ausführungsbeispiel 2 entnommen werden.

Der in Fig. 1 perspektivisch, in Fig. 2 im Schnitt und in Fig. 3 von unten gezeigte erfindungsgemäße diagnostische Teststreifen (1) weist auf einer Tragschicht (2) zwei aus aus je zwei übereinanderliegenden erfindungsgemäßen Funktionsschichten hoher Präzision (3 bis 3c) bestehende Nachweisschichten auf, die durch die Spreitauflage (6) überdeckt sind. Neben den Nachweisschichten ist die Spreitauflage (6) mittels Abstandhaltern (4,4a) und Adhäsionsschichten (5,5a) auf der Tragschicht (2) befestigt. Diese Abstandhalter können in der Praxis auch Schmelzkleberflächen oder zweiseitig klebende Bänder sein, die die Spreitauflage (6) auf der Tragschicht (2) fixieren. Idealerweise besitzen die Abstandhalter mit ihren Klebeflächen in etwa die gleiche Dicke wie die Nachweisschicht. Der hier dargestellte Aufbau weist ferner Abdeckungen (7,7a) auf, die auf der Tragschicht (2) und der Spreitauflage (6) befestigt sind. Sie sind so angeordnet, daß sie die über die Nachweisschichten hinausragenden Bereiche und einen Teil der über der Nachweisschicht liegenden Fläche der Spreitauflage (6) überdecken. Sie lassen jedoch einen Bereich über der Mitte der Nachweisschicht frei, der die Probenauftragsstelle (8) darstellt. Hierauf wird die zu untersuchende Probenflüssigkeit aufgegeben. Die linke Abdeckung (7) weist einen aufgedruckten Pfeil (9) auf, der dem Anwender zeigt, mit welchem Ende der Testträger (1) in ein Meßgerät gelegt oder geschoben werden soll. Das Positionierloch (10) dient dazu, den Teststreifen im Falle einer apparativen, beispielsweise einer reflektionsphotometrischen Vermessung an einer genau vorbestimmten Stelle des Apparates festzuhalten. Dies kann dadurch geschehen, daß beispielsweise ein Stift in das Positionierloch (10) hineinragt und so den Testträger (1) an einer vorbestimmten Stelle festhält.

Die selbstverständlich vorhandene Befestigung der Nachweisschichten und der Abstandshalter auf dem Träger, die ebenfalls über Adhäsionsschichten erfolgen kann, ist in den Figuren nicht dargestellt.

Die Figur 3 zeigt die Unterseite des erfindungsgemäßen Teststreifens mit der im Träger (2) angebrachten Positionierungsbohrung (10), der runden Beobachtungsöffnung (11), sowie der rechteckigen Meßöffnung (12), durch die die Nachweisschicht inspiziert und vermessen werden kann.

Die Figur 4 zeigt zur Verdeutlichung einen vergrößerten Ausschnitt aus der Figur 2. Auf der Tragschicht (2) sind unmittelbar aneinandergrenzend zwei aus je zwei übereinanderliegenden erfindungsgemäßen Funktionsschichten hoher Präzision (3 bis 3c) und einer die Funktionsschichten tragenden Basis-Folienschicht (13) bestehende Nachweisschichten angebracht, die durch die Spreitauflage (6) überdeckt sind. Neben den Nachweisschichten ist die Spreitauflage (6) mittels Abstandhaltern (4,4a) und Adhäsionsschichten (5,5a) auf der Tragschicht (2) befestigt. Ferner erkennt man Teile der Abdeckungen (7,7a), die auf der Spreitauflage (6) befestigt sind.

Zur Herstellung eines Testelements eines erfindungsgemäßen Testträgers werden zunächst die zum Aufbau des Testelements erforderlichen Funktionsschichten wie oben beschrieben auf einer Unterlage erzeugt. Umfaßt das Testelement zwei Funktionsschichten, so werden diese jeweils nacheinander aus einer homogenen Dispersion der genannten Bestandteile hergestellt. Hierzu verwendet man als Unterlage für das Ausformen der Beschichtungsmasse für die erste Filmschicht die transparente Folie. Nach Aufbringen der Beschichtungsmasse für die erste Filmschicht in einer bestimmten Schichtdicke wird die Schicht getrocknet. Danach wird auf diese Schicht die Beschichtungsmasse für die zweite Schicht ebenfalls in einer dünnen Schichtdicke aufgebracht und getrocknet. Nach dem Trocknen sollte die Dicke der ersten und zweiten Filmschicht zusammen maximal 0,2 mm, bevorzugt maximal 0,12 mm, besonders bevorzugt maximal 0,08 mm betragen.

In analoger Weise können auch Nachweiselemente mit mehr als zwei Funktionsschichten hergestellt werden.

In einem erfindungsgemäßen Teststreifen müssen nicht alle Funktionsschichten erfindungsgemäße Schichten hoher Präzision sein. Vielmehr können Schichten, die nur Hilfsfunktionen haben auch herkömmliche Schichten ohne einen Gehalt an Verbindungen der Formeln I, II und/oder III sein. In der Regel ist es jedoch vorteilhaft, alle Schichten erfindungsgemäß in hoher Präzision herzustellen.

Die Befestigung der Nachweiselemente auf dem Trägermaterial kann nach dem Fachmann aus der Testträgertechnologie bekannten Methoden erfolgen. Beispielsweise kann die Befestigung mittels Schmelzkleber oder Kaltkleber erfolgen. Als vorteilhaft haben sich auch doppelseitig klebende Streifen erwiesen.

Die über dem Testelement liegende Spreitauflage ist mit ihm nicht verbunden, sondern liegt nur lose auf, steht aber möglichst vollflächig mit ihm in Berührung. Normalerweise wird die Auflage in Richtung der Längsachse des Teststreifens gesehen, in einem vor und einem hinter dem Testfeld gelegenen Flächenbereich auf dem Träger fixiert. Vorteilhaft ist es, wenn das Mittel, das zur Befestigung der Auflage auf der Tragschicht dient, etwa die gleiche Dicke wie die Nachweisschicht(en) hat. Es dient dann quasi als Abstandshalter, um die erfindungsgemäße Auflage auch außerhalb des Bereiches der Nachweisschicht(en) insgesamt auf einer durchgehenden Fläche eben zu halten.

Die Spreitauflage hat die Aufgabe die Probenflüssigkeit gleichmäßig und möglichst schnell dem Nachweiselement zuzuführen bzw. sie gegebenenfalls auf alle vorhandenen Nachweiselemente gleichmäßig zu verteilen. Gegebenenfalls kommt der Spreitauflage darüberhinaus die Aufgabe zu, Überschüsse der Analytprobe von dem Testelement abzuleiten. Auf diese Art und Weise ist die bei Anwesenheit eines Analyts sich einstellende Signalintensität unabhängig von der Menge und der Dauer des Kontaktes der Probenflüssigkeit mit der Nachweisschicht. Die Farbe, die sich nach Beendung der Nachweisreaktion, üblicherweise innerhalb weniger Sekunden bis weniger Minuten eingestellt hat, bleibt so für die Messung unverändert. Sie wird lediglich durch die Stabilität des farbgebenden Systems bestimmt, nicht aber beispielsweise durch Analyt, der aus überschüssiger Flüssigkeit in die Nachweisschicht nachdiffundiert. Falsch positive Resultate werden so ebenfalls vermieden und eine quantitative Analytbestimmung ermöglicht.

Um dieser Zusatzaufgabe gerecht werden zu können, ist die Spreitauflage gegebenenfalls größer als das abzudeckende Testelement, damit ein Probenüberschuß von den das Testelement "überragenden" Teilen der Auflage aufgenommen werden kann.

Ferner ist es wichtig, daß die Befestigung der Auflage auf der Tragschicht so erfolgt, daß von der Nachweisschicht ein kapillaraktiver Flüssigkeitstransport in den Teil der Auflage möglich ist, der auf der Tragschicht befestigt ist. Beispielsweise kann die Befestigung mittels Schmelzkleber oder Kaltkleber erfolgen. Dabei ist eine punktuelle oder gerasterte Verklebung vorteilhaft, da der kapillaraktive Flüssigkeitstransport in diesem Fall besonders gut möglich ist. Dieser kapillaraktive Flüssigkeitstransport muß insbesondere dann möglich sein, wenn die Nachweisschicht mit Flüssigkeit gesättigt ist. Für die Verarbeitung ganz besonders geeignet haben sich Klebebänder mit Natur- oder Synthesekautschuk erwiesen.

Eine Spreitauflage, die sich sehr gut für die Kombinationsaufgabe eignet, Probenflüssigkeit schnell und gleichmäßig über das Testelement zu verteilen, und Überschüsse derselben in seitliche Speicherbereiche abzuleiten, besteht beispielsweise aus einem schiebefesten Gewebe mit Leinwandbindung und einer Maschenweite von 200 bis 350 µm, vorzugsweise 250 bis 300 µm, aus Monofilamenten mit einem Titer von 150 bis 300 dtex, vorzugweise 200 bis 250 dtex.

Je nach der Art der auszuführenden Reaktion, des nachzuweisenden Analyts, der Konstruktion des Teststreifens und der Art der Probensubstanz kann das Spreitmaterial so gewählt werden, daß es entweder eine hohe Durchlässigkeit hat, d.h. die Probenflüssigkeit dem Testfeld besonders schnell zuführt, oder daß seine Spreitwirkung überwiegt, um z.B. Probenmaterial sicher auf zwei oder mehrere nebeneinander liegende Testfelder zu verteilen. In jedem Fall wird man Durchlässigkeit und Spreitwirkung so aufeinander abstimmen, daß selbst bei hoher Durchlässigkeit ein Probenüberschuß sicher in die nicht mehr mit dem (den) Testfeld(ern) in Berührung stehenden Teilen der Auflage abgeleitet werden kann.

Ein großer Vorteil eines so konstruierten Testträgers besteht darin, daß kein genau vorbestimmtes Volumen einer Probenflüssigkeit auf den Testträger aufgegeben werden muß, sondern daß für das Aufgabevolumen ein großer Spielraum zur Verfügung steht. Überschüssige Flüssigkeit wird, wie bereits vorstehend erwähnt, durch die über die Nachweisschicht hinausragende Spreitauflage von der Nachweisschicht weggeleitet. Weil überschüssige Flüssigkeit von der Nachweisschicht weggeleitet wird, wird auch hygienischen Gesichtspunkten Rechnung getragen. Ein Herabtropfen von Flüssigkeit aus dem Testträger oder ein Kontakt von Flüssigkeit beispielsweise mit Teilen eines Gerätes, in das der Testträger zur apparativen Auswertung gelegt wird, wird zuverlässig vermieden. Dies ist insbesondere bei der Untersuchung von Blut oder von Blut abgeleiteten Proben, wie Plasma oder Serum, ein sehr wichtiger Aspekt.

Die Reagenzien, die für den Nachweis bestimmter Analyte erforderlich sind, und eventuell zweckmäßige Hilfs- und/oder Zusatzstoffe können in einer einzelnen erfindungsgemäßen Funktionsschicht vereinigt sein.

Es sind jedoch auch Fälle vorstellbar, für die es vorteilhafter ist, die Reagenzien und eventuell einzusetzende Hilfs- und/oder Zusatzstoffe auf mehrere saug- oder quellfähige Materialschichten zu verteilen, die dann übereinander, sich vollflächig berührend, vorzugsweise miteinander fest verbunden, angeordnet sind. Die verwendeten Begriffe Testelement, Testfeld, Nachweiselement und Nachweisschicht soll sowohl solche Fälle umfassen, bei denen sich die Reagenzien entweder nur in oder auf einer Schicht oder in zwei oder noch mehr, wie vorstehend beschrieben, angeordneten Schichten befinden.

Außerdem können die Nachweiselemente auch eine Schicht enthalten, die Plasma oder Serum aus Vollblut abzutrennen in der Lage ist, wie beispielsweise ein Glasfaservlies, wie es zum Beispiel aus EP-B-0 045 476 bekannt ist. Eine oder mehrere solcher Abtrennschichten kann über einer oder mehreren Schichten, die Nachweisreagenzien tragen, liegen. Auch ein solcher Aufbau soll von den Begriffen Testelement, Testfeld, Nachweiselement Nachweisschicht umfaßt sein.

Ganz besonders bevorzugt wird in einem erfindungsgemäßen diagnostischen Testträger ein Testelement eingesetzt, das aus zwei Funktionsschichten aufgebaut ist. Dieses Testfeld umfaßt eine transparente Folie, auf die in dieser Reihenfolge eine erste und eine zweite Funktionsschicht übereinanderliegend aufgebracht sind. Wesentlich ist, daß die auf der transparenten Folie befindliche erste Schicht im feuchten Zustand bedeutend weniger lichtstreuend ist als die darüberliegende zweite Schicht. Die nicht beschichtete Seite der transparenten Folie wird als Nachweisseite bezeichnet und die Seite der zweiten Schicht, die der Seite gegenüberliegt, mit der die zweite Schicht auf der ersten aufliegt, wird als Probenaufgabenseite bezeichnet.

Die beiden Funktionsschichten können aus Beschichtungsmassen hergestellt werden, die den gleichen polymeren Filmbildner enthalten oder sie können aus Beschichtungsmassen erzeugt werden, die unterschiedliche polymere Filmbildner enthalten.

Sofern besondere Testaufgaben und/oder Testbedingungen vorliegen, wie z.B. bei der Bestimmung von Glukose in Vollblut, ist es zweckmäßig, die Schichten so auszubilden, daß sie außer guter Erythrozytenseparation auch optische Merkmale aufweisen, die die Beobachtung der Nachweisreaktion erleichtern und die Exaktheit der Beurteilung und der meßtechnischen Erfassung verbessern.

Hierzu enthält die erste Schicht zweckmäßigerweise ein Quellmittel und gegebenenfalls einen schwach lichtstreuenden Füllstoff, die zweite Schicht ein Quellmittel und wenigstens ein stark lichtstreuendes Pigment. Da die schwach lichtstreuenden Füllstoffe und die stark lichtstreuenden Pigmente wesentlich für die optischen Eigenschaften der Filmschichten verantwortlich sind, besitzen die erste und die zweite Filmschicht unterschiedliche Füllstoffe und Pigmente.

Die erste Funktionsschicht soll entweder keine oder solche Füllstoffe enthalten, deren Brechungsindex nahe beim Brechungsindex von Wasser liegt. Als besonders geeignet hierfür haben sich Siliziumdioxid, Silikate und Aluminiumsilikate erwiesen. Ein Natriumaluminiumsilikat mit dem Handelsnamen Transpafill® (Fa. Degussa, Frankfurt(M)) ist besonders bevorzugt.

Die zweite Schicht soll möglichst stark lichtstreuend sein. Idealerweise liegt der Brechungsindex der Pigmente in der zweiten Filmschicht mindestens bei 2,5. Daher wird vorzugsweise Titandioxid eingesetzt. Teilchen mit einem mittleren Durchmesser von etwa 0,2 bis 0,8 μm haben sich als besonders vorteilhaft erwiesen.

Über der Spreitauflage des erfindungsgemäßen diagnostischen Testträgers kann zweckmäßigerweise eine inerte Abdeckung aus probenundurchlässigem, in der Regel wasserundurchlässigem und nicht saugfähigem Material, so angeordnet werden, daß der Bereich der Spreitauflage außerhalb der Nachweisschicht abgedeckt ist. Idealerweise ragt die Abdeckung auch noch in den Bereich der Nachweisschicht hinein, läßt jedoch auf jeden Fall einen mittleren Teil der erfindungsgemäßen Auflage, die die Nachweisschicht bedeckt, frei. Dieser freie Teil der Auflage wird als Probenauftragsstelle bezeichnet. Zweckmäßigerweise beträgt die von der inerten Abdeckung für den Probenauftrag freigelassene Strecke 2 bis 5 mm. Die Probenauftragsstelle befindet sich vorzugsweise über der Lochung in der Tragschicht, durch die eine Signalbildung in der Nachweisschicht beobachtet werden kann.

Eine Probenauftragsstelle kann besonders einfach durch eine Abdeckung mittels zweier bandförmiger Kunststoffolien erreicht werden, die einen bandartigen Bereich der die Nachweisschicht überdeckenden Spreitauflage frei lassen. Wenn 2 oder mehr Probenauftragsstellen vorgesehen werden, sind 3 oder mehr bandförmige Kunststoffolien zu verwenden. Die zur Abdeckung verwendeten Folien sind auf der Spreitauflage und gegebenenfalls auf der Tragschicht befestigt. Für eine solche Befestigung eignen sich Schmelzkleber, die vorzugsweise punktuell oder gerastert auf der Tragschicht oder der Unterseite der Abdeckung aufgebracht sind oder Klebebänder, wenn die Folien nicht selbst klebefähig sind. Soll für eine Abfuhr eines eventuellen Probenüberschusses gesorgt werden, so ist darauf zu achten, daß zwischen der Abdeckung und der Spreitauflage ein Kapillarspalt verbleibt, in den überschüssige Probenflüssigkeit von einer mit Flüssigkeit gesättigten Nachweisschicht aufgenommen werden kann.

Außerdem führt die Abdeckung dazu, daß von der Nachweisschicht weggeleitete überschüssige Flüssigkeit von Kontakt von außen geschützt ist und solche Flüssigkeit nicht leicht von dem Testträger herabtropfen kann.

Durch die Abdeckung von Teilen der erfindungsgemäßen Auflage und damit der Markierung der Probenauftragsstelle wird dafür Sorge getragen, daß Flüssigkeit nur an der dafür optimalen Stelle auf die Nachweisschicht gelangen kann. In Kombination mit einer Nachweisschicht, die nur wenig Flüssigkeit aufnimmt und dennoch eine intensive Signalbildung gewährleistet, wird sichergestellt, daß bereits bei sehr kleinen Probenvolumina zuverlässige Analytbestimmungen möglich sind. Dadurch, daß der erfindungsgemäße Testträger aus nur wenigen Komponenten besteht, die einfach und schnell zusammenfügbar sind, ist er sehr preiswert herzustellen.

Zur Durchführung eines Verfahrens zur Bestimmung von Analyt in flüssiger Probe mit Hilfe eines erfindungsgemäßen diagnostischen Testträgers wird Probenflüssigkeit auf die dem Nachweiselement abgewandten Seite der Spreitauflage aufgegeben, idealerweise so viel, daß die durch die Spreitauflage gelangende Flüssigkeit die Nachweisschicht vollständig sättigt. Als Probenflüssigkeit kommen insbesondere Körperflüssigkeiten wie Blut, Plasma, Serum, Urin, Speichel etc. in Frage. Blut oder von Blut abgeleitete Flüssigkeiten wie Plasma oder Serum sowie Urin sind besonders bevorzugte Probenflüssigkeiten. Überschüssige Flüssigkeit wird durch die Spreitauflage von dem Nachweiselement in den über das Nachweiselement hinausragenden Bereich der Auflage weggeführt. In dem Nachweiselement kann dann bei Anwesenheit des zu bestimmenden Analyts ein Signal nachgewiesen werden. Vorteilhafterweise handelt es sich bei einem solchen Signal um eine Farbänderung, worunter sowohl Farbbildung, Farbverlust als auch Farbumschlag verstanden wird. Zur Bestimmung des in der Probenflüssigkeit nachzuweisenden Analyts ist in dem erfindungsgemäßen diagnostischen Testträger das Nachweiselement, zumindest aber sind die Reaktionsbezirke, das heißt, Reagenz tragende Bereiche des Nachweiselements, die auf Signalbildung hin beobachtet und vermessen werden können, durch die Tragschicht sichtbar. Dies kann, wie oben bereits ausgeführt, dadurch erreicht werden, daß die Tragschicht transparent ist oder unter dem Nachweiselement gelocht ist.

Die Intensität der Farbänderung ist ein Maß für die Menge an Analyt in der untersuchten flüssigen Probe. Sie kann visuell oder mit Hilfe eines Gerätes, meistens reflektionsphotometrisch, quantitativ ausgewertet werden, wobei in Vorversuchen geschaffene Eichkurven benutzt werden können. Alternativ kann auch eine direkte Anzeige des Analytgehalts über die Geräte-Software bewerkstelligt werden.

Besonders bevorzugt sind auch solche Ausführungsformen der vorliegenden Erfindung, die eine Kombination mehrerer bevorzugter Merkmale aufweisen.

Die folgenden Ausführungsbeispiele veranschaulichen die Herstellung erfindungsgemäßer Funktionsschichten und Teststreifen und zeigen deren signifikante Überlegenheit gegenüber herkömmlichen Produkten.

**Beispiel 1.**

Das Beispiel veranschaulicht Beschichtungsmassen zur Herstellung erfindungsgemäßer Funktionsschichten und zeigt die Wirkung eines erfindungsgemäß einzusetzenden Netzmittels auf die Oberflächenspannung der Massen.

Mischung A.

In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser | 820,0 g |
| Natrium-N-methyl-N-oleoyl-taurat* | X g |
| Citronensäure-1-hydrat | 2,5 g |
| Calciumchlorid-2-hydrat | 0,5 g |
| Natriumhydroxid | 1,4 g |
| Xanthan gum | 3,4 g |
| Tetraethylammoniumchlorid | 2,0 g |
| N-Octanoyl-N-methyl-glucamid | 2,1 g |
| Polyvinylpyrrolidon (MG 25000) | 3,5 g |
| Transpafill( (Natrium-Aluminiumsilikat) | 62,1 g |
| Polyvinylpropionat-Dispersion (50 Gew.-% in Wasser) | 60,8 g |
| Bis-(2-hydroxyethyl)-(4-hydroximinocyclohexa-2,5-dienylidin)-ammoniumchlorid | 1,2 g |
| 2,18-Phosphormolybdänsäure-hexanatriumsalz | 16,1 g |
| Pyrrolochinolin-chinon | 32 mg |
| Glucosedehydrogenase rec. aus Acinetobacter calcoaceticus, 1,7 MU EC 1.1.99.17 | 2,4 g |

\* (®Geropon T 77, Handelsprodukt der Fa. Rhone-Poulenc Chimie)

Die Gesamtmasse wird mit NaOH auf einen pH von ca. 6 eingestellt.

Die obige Mischung wurde mit folgenden Mengen X des Geropons T 77 hergestellt:

X=0 entsprechend 0 Gew.-% Geropon
X=0,0098 entsprechend 0,001 Gew.-% Geropon
X=0,098 entsprechend 0,01 Gew.-% Geropon
X=0,29 entsprechend 0,03 Gew.-% Geropon
X=0,98 entsprechend 0,1 Gew.-% Geropon
X=2,9 entsprechend 0,3 Gew.-% Geropon

Von jeder der so hergestellten Mischungen wurde die Oberflächenspannung [mN/m] bei 20°C nach der Ringmethode nach DIN 53914 unter Verwendung eines Digitaltensiometers K10T der Fa. Krüss GmbH,Hamburg gemessen. Die Ergebnisse der Messungen sind in der Tabelle 1 angegeben.

Mischung B.

In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser | 579,7 g |
| Natrium-N-methyl-N-oleoyl-taurat | X g |
| Natriumhydroxid | 3,4 g |
| Gantrez( (Methylvinylether-maleinsäure-Copolymer) | 13,8 g |
| N-Octanoyl-N-methyl-glucamid | 3,6 g |
| Tetraethylammoniumchlorid | 9,7 g |
| Polyvinylpyrrolidon (MG 25000) | 20,2 g |
| Titandioxid | 177,1 g |
| Kieselgur | 55,3 g |
| Polyvinylpropionat-Dispersion (50 Gew.-% in Wasser | 70,6 g |
| 2,18-Phosphormolybdänsäure-hexanatriumsalz | 44,3 g |
| Kaliumhexacyanoferrat (III) | 0,3 g |

Auch diese Mischung wurde mit den oben für X angegebenen Mengen Geropon hergestellt. Anschließend wurden wie bei Mischung A die Oberflächenspannungen gemessen und in der Tabelle 1 angegeben.

Tabelle 1

| Oberflächenspannung der Beschichtungsmassen A und B in Abhängigkeit von der Geropon-Konzentration. | | |
|---|---|---|
| Geropon-Konzentration [%] | Oberflächenspannung A | Oberflächenspannung B |
| 0 | 48,1 | 58,1 |
| 0,001 | 46,2 | 56,4 |
| 0,01 | 43,8 | 50,7 |
| 0,03 | 41,7 | 41,5 |
| 0,1 | 40,4 | 39,3 |
| 0,3 | 38,5 | 39,3 |

**Beispiel 2.**

Herstellung erfindungsgemäßer Teststreifen.

Auf eine bandförmige, 50 mm breite Titandioxid-haltige Polyester-Tragschicht wird parallel im Abstand von 18,6 mm (gemessen zur linken Kante des Klebebandes) zu seiner linken Kante ein 5 mm breites doppelseitiges Klebeband (Polyesterträger und Synthesekautschuk-Kleber) aufgebracht. Aus diesem Verbund werden mit einem Abstand von 6 mm jeweils zwei Löcher, ein Positionierungsloch und ein Inspektions- und Meßloch, ausgestanzt, deren Mittelpunkte auf einer senkrecht zur Längachse der Trägerstreifens liegenden Geraden liegen. Das erste Loch, das Positionierungsloch, ist kreisrund, hat einen Durchmesser von 2,6 mm und sein Mittelpunkt hat von der linken Kante des Trägerstreifens einen Abstand von 4 mm. Das zweite Loch ist ebenfalls rund mit einem Durchmesser von 4 mm. Der Mittelpunksabstand des zweiten Loches von der linken Kante des Trägerstreifens beträgt 21 mm.

Danach wird das Schutzpapier des doppelseitigen Klebebands abgezogen.

Zur Herstellung einer Nachweisschicht, die aus 2 Filmschichten aufgebaut ist, wird so vorgegangen:

In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser | 820,0 g |
| Natrium-N-methyl-N-oleoyl-taurat | 0,29 g |
| Citronensäure-1-hydrat | 2,5 g |
| Calciumchlorid-2-hydrat | 0,5 g |

(fortgesetzt)

| | |
|---|---|
| Natriumhydroxid | 1,4 g |
| Xanthan gum | 3,4 g |
| Tetraethylammoniumchlorid | 2,0 g |
| N-Octanoyl-N-methyl-glucamid | 2,1 g |
| Polyvinylpyrrolidon (MG 25000) | 3,5 g |
| Transpafill( (Natrium-Aluminiumsilikat) | 62,1 g |
| Polyvinylpropionat-Dispersion (50 Gew.-% in Wasser) | 60,8 g |
| Bis-(2-hydroxyethyl)-(4-hydroximinocyclohexa-2,5-dienylidin)-ammoniumchlorid | 1,2 g |
| 2,18-Phosphormolybdänsäure-hexanatriumsalz | 16,1 g |
| Pyrrolochinolin-chinon | 32 mg |
| Glucosedehydrogenase rec. aus Acinetobacter calcoaceticus, 1,7 MU EC 1.1.99.17 | (2,4 g) |
| 1-Hexanol | 1,6 g |
| 1-Methoxy-2-propanol | 20,4 |

Die Gesamtmasse wird mit NaOH auf einen pH-Wert von ca. 6 eingestellt und dann mit einem Flächengewicht von 89 g/qm auf eine 125 µ dicke Polycarbonatfolie aufgetragen und getrocknet.

In einem Becherglas werden die folgenden Komponenten als Reinsubstanzen oder in Form von Stammlösungen in folgender Zusammensetzung zusammengegeben und durch Rühren gemischt:

| | |
|---|---|
| Wasser | 579,7 g |
| Natrium-N-methyl-N-oleoyl-taurat | 0,29 g |
| Natriumhydroxid | 3,4 g |
| Gantrez( (Methylvinylether-maleinsäure-Copolymer) | 13,8 g |
| N-Octanoyl-N-methyl-glucamid | 3,6 g |
| Tetraethylammoniumchlorid | 9,7 g |
| Polyvinylpyrrolidon (MG 25000) | 20,2 g |
| Titandioxid | 177,1 g |
| Kieselgur | 55,3 g |
| Polyvinylpropionat-Dispersion (50 Gew.-% in Wasser) | 70,6 g |
| 2,18-Phosphormolybdänsäure-hexanatriumsalz | 44,3 g |
| Kaliumhexacyanoferrat (III) | 0,3 g |
| 1-Hexanol | 1,6 g |
| 1-Methoxy-2-propanol | 20,4 |

Die Gesamtmasse wird mit NaOH auf einen pH von ca. 6 eingestellt und dann als zweite Schicht mit einem Flächengewicht von 104 g/qm auf die wie vorstehend unter A. beschriebene beschichtete Polycarbonatfolie aufgetragen und getrocknet.

B.4) Ein 5 mm breiter Streifen der so hergestellten Nachweisschicht wird mit der Folienseite auf das gestanzte doppelseitige Klebeband auf der Tragschicht passgenau aufgeklebt.

Direkt an die Nachweisschicht angrenzend werden auf beiden Seiten doppelseitige Klebebänder als Abstandshalter auf die Trägerfolie aufgeklebt. Im vorliegenden Beispiel ist ein Abstandshalter 6 mm und der andere 9 mm breit. Danach wird die Schutzfolie der beiden doppelseitigen Klebebänder abgezogen.

Auf diesen Verbund wird ein 20 mm breiter Streifen des in Abschnitt A hergestellten Spreitvlieses aufgelegt und durch Anpressen verklebt.

Es werden zwei einseitige Klebebänder als Abdeckungen so auf das Spreitvlies aufgeklebt, daß die Abstandshalter ganz abgedeckt werden und wenigstens noch eine geringfügige Überlappung mit dem Reaktivbezirk stattfindet. Damit ist die Bandware fertiggestellt.

Die Bandware wird so in 6 mm breite Testträger geschnitten, daß das Meßloch im Testträger mittig liegt.

## Beispiel 3

Die Herstellung von Teststreifen gemäß Beispiel 2 wird wiederholt mit dem Unterschied, daß der Zusatz der 0,29 g Natrium-N-methyl-N-oleoyl-taurat weggelassen wurde.

Signifikante Unterschiede zwischen den Beschichtungsmassen der Beispiele 2 und 3 zeigten sich bereits beim Aufrakeln auf die Polycarbonat-Basisfolie. Mit den Massen des Beispiels 2 wurde eine fehlstellenfreie und wesentlich glattere Beschichtung erzielt als mit den Massen des Beispiels 3.

Außerdem konnte bei Verwendung der Massen des Beispiels 2 eine praktisch fehlstellenfreie Erstbeschichtung auch ohne Plasmavorbehandlung der Polycarbonatfolie gefertigt werden.

**Beispiel 4**

Wirkung des erfindungsgemäßen Netzmittelzusatzes auf die Präzision der Funktionsschichten der gemäß Beispiel 2 hergestellten Teststreifen.

Unter Verwendung von gemäß Beispiel 2 hergestellten erfindungsgemäßen und von gemäß Beispiel 3 hergestellten herkömmlichen Teststreifen und Benutzung von ®GLUCOTREND-Geräten wurde in Vollblut mit vier verschiedenen, vorgegebenen Glucosekonzentrationen der Glucosegehalt bestimmt. Es zeigte sich, daß die Reaktionsgeschwindigkeit und die Farbtiefe (% Remission) durch den Zusatz des erfindungsgemäßen Netzmittels nicht beeinflußt wurden. Es wurden für jede Glucosekonzentration und die Varianten ohne und mit Geropon T77 10 Meßserien mit je 10 Einzelmessungen (n=10, N=100) ausgeführt. Hieraus wurden für jede Glucosekonzentration und die Varianten ohne und mit Geropon T77 10 VK-Werte berechnet, deren Mediane in der Tabelle 2 aufgeführt wurden. (Der VK-Wert ist definiert als die relative Standardabweichung

$$VK = Standardabweichung / Mittelwert$$

und wird in % angegeben.)

Tabelle 2

| Präzision von Glucosebeschichtungen ohne und mit 0,03 Gew.-% Geropon in der nassen Beschichtungsmasse. | | |
|---|---|---|
| Glucosekonzentration [mg/dl] | VK ohne Geropon [%] | VK mit Geropon [%] |
| 50 | 4,1 | 3,2 |
| 100 | 2,8 | 2,3 |
| 200 | 3,0 | 2,4 |
| 400 | 3,4 | 3,0 |

Die Tabelle zeigt, daß bei Einsatz der erfindungsgemäßen Teststreifen, hergestellt gemäß Beispiel 2, die Mediane der VK-Werte um durchschnittlich 0,5 % (absolut) geringer ausfielen als bei Verwendung herkömmlicher Teststreifen, hergestellt gemäß Beispiel 3. Dies entspricht einer Verbesserung der Zuverlässigkeit der Meßergebnisse um durchschnittlich 18%, was auf die durch den erfindungsgemäßen Zusatz von Natrium-N-methyl-N-oleoyl-taurat verbesserte Präzision der Funktionsschichten der Teststreifen zurückzuführen ist.

**Patentansprüche**

**1.** Skelettfreie Funktionsschicht hoher Präzision umfassend einen Film aus einem natürlichen oder synthetischen filmbildenden Polymer (Filmbildner), einer oder mehreren, die Funktion der Schicht ermöglichenden Verbindungen umfassend Reagenzien zum qualitativen Nachweis oder zur quantitativen Bestimmung von Analyten, gegebenenfalls Hilfs- und/oder Zusatzstoffen, **dadurch gekennzeichnet, dass** die Schicht ein Netzmittel der Formel I, II und/oder III enthält,

$$R^1\text{-CO-N}(R^2)\text{-CH}_2\text{-COOMe} \qquad (I)$$

$$R^1\text{-CO-N}(R^2)\text{-CH}_2\text{- CH}_2\text{-SO}_3\text{Me} \qquad (II)$$

$$HO_2C\text{-CH}_2\text{-CH}_2\text{-CH(NH-COR}^1)\text{-CO}_2\text{Me} \qquad (III)$$

worin R$^1$ ein vorzugsweise geradkettiger oder ein wenig verzweigter aliphatischer Rest mit 9 bis 23 C-Atomen, insbesondere mit 11 bis 19 C-Atomen ist, der gesättigt ist oder eine bis drei Doppelbindungen aufweist, R$^2$ ein vorzugsweise geradkettiger oder wenig verzweigter Alkylrest mit 1 bis 8, vorzugsweise 1 bis 4, C-Atomen ist, und

Me für Wasserstoff- oder Metallatom steht.

2. Skelettfreie Funktionsschicht gemäß Anspruch 1, **dadurch gekennzeichnet, daß** R$^1$ die aliphatische Kette der Laurinsäure, der Myristinsäure, der Palmitinsäure, der Stearinsäure der Palmitoleinsäure, der Oleinsäure, der Linolsäure, der Linolensäure oder deren Isomeren und

   R$^2$ Methyl oder Ethyl ist.

3. Skelettfreie Funktionsschicht gemäß mindestens einem der Ansprüche 1 und 2, **dadurch gekennzeichnet, daß** R$^1$ für eine Menge von Alkylresten, in dem die einzelnen Alkylreste bezüglich ihrer Struktur und ihres Anteils in der Mischung der Struktur und dem Anteil ihres Vorkommens in natürlichen Fetten entsprechen, steht.

4. Skelettfreie Funktionsschicht gemäß mindestens einem der Ansprüche 1 bis 3, **dadurch gekennzeichnet, daß** R$^1$-CO- Oleoyl, Kokoyl -oder der Talgfettsäurerest und R$^2$ Methyl ist.

5. Skelettfreie Funktionsschicht gemäß mindestens einem der Ansprüche 1 bis 4, **dadurch gekennzeichnet, daß** Me ein solches Metallatom ist, daß die Verbindungen der Formel I II, und III wasserlöslich sind.

6. Skelettfreie Funktionsschicht gemäß mindestens einem der Ansprüche 1 bis 5, **dadurch gekennzeichnet, daß** Me ein Alkalimetall, vorzugsweise Natrium oder Kalium ist.

7. Skelettfreie Funktionsschicht gemäß mindestens einem der Ansprüche 1 bis 6, **dadurch gekennzeichnet, daß** das Netzmittel eine Mischung von Verbindungen der Formel I, II und/oder III ist.

8. Skelettfreie Funktionsschicht gemäß mindestens einem der Ansprüche 1 bis 7, **dadurch gekennzeichnet, dass** sie insgesamt 0,0075 bis 2,5 Gew.-%, vorzugsweise 0,01 bis 2,0 Gew.-%, Netzmittel der Formel I, II, und/oder III, worin die Reste R$^1$, R$^2$, und Me die oben angegebene Bedeutung haben, enthält.

9. Skelettfreie Funktionsschicht gemäß mindestens einem der Ansprüche 1 bis 8, **dadurch gekennzeichnet, dass** sie Teil eines diagnostischen Teststreifens ist.

10. Verfahren zur Herstellung von skelettfreien Funktionsschichten hoher Präzision gemäß einem der Ansprüche 1 bis 9 durch Beschichten einer Unterlage mit einer flüssigen oder pastösen Beschichtungsmasse aus einem flüssigen Lösungs- oder Dispergiermittel, einer Lösung, einer Dispersion oder einem redispergierbaren oder löslichen Präparat eines natürlichen oder synthetischen filmbildenden Polymers (Filmbildner), einer oder mehreren, die Funktion der Schicht ermöglichenden Verbindungen und gegebenenfalls Hilfs- und/oder Zusatzstoffen, und anschließendes Entfernen des flüssigen Lösungsoder Dispergiermittels, **dadurch gekennzeichnet, dass** die flüssige oder pastöse Mischung ein Netzmittel der Formel I, II und/oder III enthält, worin die Reste R$^1$, R$^2$, und Me die in den Ansprüchen 1 bis 6 angegebenen Bedeutungen haben.

11. Verfahren gemäß Anspruch 10, **dadurch gekennzeichnet, dass** die Unterlage bereits eine oder mehrere Funktionsschichten trägt.

12. Verfahren gemäß mindestens einem der Ansprüche 10 und 11, **dadurch gekennzeichnet, dass** die flüssige oder pastöse Beschichtungsmasse insgesamt 0,0075 bis 2,5 Gew.-%, vorzugsweise 0,01 bis 2,0 Gew.-%, bezogen auf das Gewicht aller Bestandteile der Beschichtungsmasse mit Ausnahme des Wassers, von Netzmitteln der Formel I, II, und/oder III, worin die Reste R$^1$, R$^2$, und Me die oben angegebene Bedeutung haben, enthält.

13. Teststreifen mit einem flexiblen flächenförmigen Träger, auf dem in einem Testbereich ein oder mehrere Testfelder nebeneinander angeordnet sind, die jeweils eine oder mehrere übereinanderliegende Funktionsschichten umfassen, und gegebenenfalls durch eine Auflage aus einem Spreitmaterial abgedeckt sind, die **dadurch gekennzeichnet sind, dass** mindestens eine der Funktionsschichten eine erfindungsgemäße skelettfreie Funktionsschicht hoher Präzision gemäß einem der Ansprüche 1 bis 9 ist.

14. Teststreifen gemäß Anspruch 13, **dadurch gekennzeichnet, dass** das Testfeld eine Transparente Folie und eine erste und eine zweite darauf angebrachte übereinanderliegende Funktionsschichten umfasst,
wobei die sich auf der transparenten Folie befindliche erste Schicht im feuchten Zustand wesentlich weniger lichtstreuend ist als die darüberliegende zweite Schicht und wobei die Seite der Folie, die der Folienseite gegenüberliegt, auf der die erste Schicht aufgebracht ist, die Nachweisseite und die Seite der zweiten Schicht, die der Seite gegenüberliegt, mit der die zweite Schicht auf der ersten Schicht aufliegt, die Probenaufgabeseite ist.

**15.** Teststreifen gemäß mindestens einem der Ansprüche 13 und 14, **dadurch gekennzeichnet, dass** die Dicke der ersten und zweiten Funktionsschicht im trockenen Zustand zusammen maximal 0,20 mm, bevorzugt maximal 0,12 mm, besonders bevorzugt maximal 0,08 mm beträgt.

**16.** Teststreifen gemäß mindestens einem der Ansprüche 13 bis 15, **dadurch gekennzeichnet, dass** die Tragschicht ein Loch enthält, über dem eine Nachweisschicht mit mehreren nebeneinander befindlichen Reaktionsbezirken angeordnet ist.

**17.** Verwendung eines diagnostischen Teststreifens gemäß einem der Patentansprüche 13 bis 16 zur Bestimmung von Analyt in einer Flüssigkeit.

**18.** Verfahren zur Bestimmung von Analyt in einer flüssigen Probe mit Hilfe eines diagnostischen Teststreifens gemäß einem der Patentansprüche 13 bis 16, wobei Probenflüssigkeit auf die Probenaufgabestelle aufgegeben wird und die Nachweisschicht(en) auf die Signalbildung hin beobachtet wird (werden), wobei die Signalbildung ein Maß für die Anwesenheit bzw. Menge an Analyt in der untersuchten flüssigen Probe darstellt.

**Claims**

**1.** Skeleton-free functional layer of high precision comprising a film composed of a natural or synthetic film-forming polymer (film former), one or several compounds enabling the function of the layer comprising reagents for the qualitative detection or quantitative determination of analytes, optionally auxiliary substances and/or additives, wherein the layer contains a wetting agent of formula I, II and/or III

$$R^1\text{-CO-N}(R^2)\text{-CH}_2\text{-COOMe} \tag{I}$$

$$R^1\text{-CO-N}(R^2)\text{-CH}_2\text{-CH}_2\text{-SO}_3\text{Me} \tag{II}$$

$$HO_2C\text{-CH}_2\text{-CH}_2\text{-CH(NH-COR}^1)\text{-CO}_2\text{Me} \tag{III}$$

in which $R^1$ is a preferably straight-chained or slightly branched aliphatic residue with 9 to 23 C atoms, in particular with 11 to 19 C atoms which is saturated or has one to three double bonds,
$R^2$ is a preferably straight-chained or a slightly branched alkyl residue with 1 to 8, preferably 1 to 4 C atoms and Me represents hydrogen or a metal atom.

**2.** Skeleton-free functional layer as claimed in claim 1, wherein $R^1$ is the aliphatic chain of lauric acid, myristic acid, palmitic acid, stearic acid, palmitoleic acid, oleic acid, linoleic acid, linolenic acid or isomers thereof and $R^2$ is methyl or ethyl.

**3.** Skeleton-free functional layer as claimed in at least one of the claims 1 and 2, wherein $R^1$ represents a quantity of alkyl residues in which the structure and proportion of the individual alkyl residues in the mixture corresponds to their structure and abundance in natural fats.

**4.** Skeleton-free functional layer as claimed in at least one of the claims 1 to 3, wherein $R^1$-CO is oleoyl, cocoyl or a tallow fatty acid residue and $R^2$ is methyl.

**5.** Skeleton-free functional layer as claimed in at least one of the claims 1 to 4, wherein Me is such a metal atom that the compounds of formulae I, II and III are water-soluble.

6. Skeleton-free functional layer as claimed in at least one of the claims 1 to 5, wherein Me denotes an alkali metal, preferably sodium or potassium.

7. Skeleton-free functional layer as claimed in at least one of the claims 1 to 6, wherein the wetting agent is a mixture of compounds of formulae I, II and/or III.

8. Skeleton-free functional layer as claimed in at least one of the claims 1 to 7, wherein it contains a total of 0.0075 to 2.5 % by weight, preferably 0.01 to 2.0 % by weight wetting agent of formulae I, II and/or III in which the residues $R^1$, $R^2$ and Me have the meanings stated above.

9. Skeleton-free functional layer as claimed in at least one of the claims 1 to 8, wherein it is part of a diagnostic test strip.

10. Process for the production of skeleton-free functional layers of high precision as claimed in one of the claims 1 to 9 by coating a support with a liquid or paste-like coating composition composed of a liquid solvent or dispersant, a solution, a dispersion or a redispersible or soluble preparation of a natural or synthetic film forming polymer (film former), one or several compounds enabling the function of the layer and optionally auxiliary substances and/or additives and subsequently removing the liquid solvent or dispersant, wherein the liquid or paste-like mixture contains a wetting agent of formula I, II and/or III in which the residues $R^1$, $R^2$ and Me have the meanings stated in claims 1 to 6.

11. Process as claimed in claim 10, wherein the support already carries one or several functional layers.

12. Process as claimed in at least one of the claims 10 and 11, wherein the liquid or paste-like coating composition contains a total of 0.0075 to 2.5 % by weight, preferably 0.01 to 2.0 % by weight relative to the weight of all components of the coating composition with the exception of water, of wetting agents of formulae I, II and/or III in which the residues $R^1$, $R^2$ and Me have the meanings stated above.

13. Test strip composed of a flexible flat-shaped carrier on which one or several test fields are arranged next to one another in a test region which each comprises one or several functional layers which rest on top of one another and optionally are covered by an overlay made of a spreading material, wherein at least one of the functional layers is a skeleton-free functional layer of high precision as claimed in one of the claims 1 to 9.

14. Test strip as claimed in claim 13, wherein the test field comprises a transparent foil and mounted thereon a first and a second functional layer lying on top of each other, wherein the first layer located on the transparent foil scatters light considerably less in a wet state than the overlying second layer and wherein the side of the foil which is opposite to that side of the foil on which the first layer is applied is the detection side and the side of the second layer which is opposite to the side of the second layer which rests on the first is the sample application side.

15. Test strip as claimed in at least one of the claims 13 and 14, wherein the thickness of the first and second functional layer in the dry state is together at most 0.20 mm, preferably at most 0.12 mm and particularly preferably at most 0.08 mm.

16. Test strip as claimed in at least one of the claims 13 to 15, wherein the support layer contains a hole over which a detection layer is located containing several adjacent reaction zones.

17. Use of a diagnostic test strip as claimed in one of the claims 13 to 16 to determine analyte in a liquid.

18. Method for the determination of an analyte in a liquid sample with the aid of a diagnostic test strip as claimed in one of the claims 13 to 16, wherein a sample liquid is applied to the sample application site and the detection layer(s) is(are) observed for signal generation, the signal generation being a measure for the presence or the amount of analyte in the examined liquid sample.

**Revendications**

1. Couche fonctionnelle haute précision exempte de squelette comprenant un film constitué par un polymère filmogène naturel ou synthétique (agent filmogène), par un ou plusieurs composés permettant à la couche de fonctionner, comprenant des réactifs pour le décèlement qualitatif ou pour la détermination quantitative d'analytes, le cas échéant des adjuvants et/ou des additifs, **caractérisé en ce que** la couche contient un agent mouillant répondant aux formules I, II et/ou III

$$R^1\text{-CO-N}(R^2)\text{-CH}_2\text{-COOMe} \tag{I}$$

$$R^1\text{-CO-N}(R^2)\text{-CH}_2\text{-CH}_2\text{-SO}_3\text{Me} \tag{II}$$

$$HO_2C\text{-CH}_2\text{-CH}_2\text{-CH(NH-COR}^1)\text{-CO}_2\text{Me} \tag{III}$$

dans lesquelles $R^1$ représente un radical aliphatique de préférence à chaîne droite ou légèrement ramifiée, contenant de 9 à 23 atomes de carbone, en particulier de 11 à 19 atomes de carbone, qui est saturé ou qui présente de 1 à 3 liaisons doubles,
$R^2$ représente un radical alkyle de préférence à chaîne droite ou légèrement ramifiée, contenant de 1 à 8, de préférence de 1 à 4 atomes de carbone, et
Me représente un atome d'hydrogène ou un atome métallique.

2. Couche fonctionnelle exempte de squelette, selon la revendication 1, **caractérisée en ce que** $R^1$ représente la chaîne aliphatique de l'acide laurique, de l'acide myristique, de l'acide palmitique, de l'acide stéarique, de l'acide palmitoléique, de l'acide oléique, de l'acide linoléique, de l'acide linolénique ou de leurs isomères, et
$R^2$ représente un groupe méthyle ou un groupe éthyle.

3. Couche fonctionnelle exempte de squelette selon au moins une des revendications 1 et 2, **caractérisée en ce que** $R^1$ représente une quantité de radicaux alkyle, dans laquelle les radicaux alkyle individuels correspondent, en ce qui concerne leur structure et leur fraction dans le mélange, à la structure et à la fraction de leur présence dans des graisses naturelles.

4. Couche fonctionnelle exempte de squelette selon au moins une des revendications 1 à 3, **caractérisée en ce que** $R^1$-CO- représente un radical oléoyle, un radical cocoyle ou encore un radical d'acide gras de tallol et $R^2$ représente un groupe méthyle.

5. Couche fonctionnelle exempte de squelette selon au moins une des revendications 1 à 4, **caractérisée en ce que** Me représente un atome métallique tel que les composés répondant aux formules I, II et III sont hydrosolubles.

6. Couche fonctionnelle exempte de squelette selon au moins une des revendications 1 à 5, **caractérisée en ce que** Me représente un métal alcalin, de préférence le sodium ou le potassium.

7. Couche fonctionnelle exempte de squelette selon au moins une des revendications 1 à 6, **caractérisée en ce que** l'agent mouillant est un mélange de composés répondant aux formules I, II et/ou III.

8. Couche fonctionnelle exempte de squelette selon au moins une des revendications 1 à 7, **caractérisée en ce qu'**elle contient au total des agents mouillants répondant aux formules I, II et/ou III à concurrence de 0,0075 à 2,5 % en poids, de préférence de 0,01 à 2,0 % en poids, les radicaux $R^1$, $R^2$ et Me ayant la signification indiquée ci-dessus.

9. Couche fonctionnelle exempte de squelette selon au moins une des revendications 1 à 8, **caractérisée en ce qu'**elle représente une partie d'une bandelette réactive diagnostique.

10. Procédé pour la fabrication de couches fonctionnelles haute précision exemptes de squelette selon l'une quelconque des revendications 1 à 9, par enduction d'un substrat avec une matière d'enduction liquide ou pâteuse constituée par un dissolvant ou un agent de mise en dispersion liquide, une solution, une dispersion ou encore une préparation apte à être remise en dispersion ou soluble d'un polymère filmogène naturel ou synthétique (agent filmogène), un ou plusieurs composés permettant à la couche de fonctionner et le cas échéant des adjuvants et/ou des additifs, et par élimination ultérieure du dissolvant ou de l'agent de mise en dispersion liquide, **caractérisée**

**en ce que** le mélange liquide ou pâteux contient un agent mouillant répondant aux formules I, II et/ou III, les radicaux $R^1$, $R^2$ et Me ayant la signification indiquée dans les revendications 1 à 6.

**11.** Procédé selon la revendication 10, **caractérisé en ce que** le substrat porte déjà une ou plusieurs couches fonctionnelles.

**12.** Procédé selon l'une quelconque des revendications 10 et 11, **caractérisé en ce que** la matière d'enduction liquide ou pâteuse contient au total des agents mouillants répondant aux formules I, II et/ou III au total à concurrence de 0,0075 à 2,5 % en poids, de préférence de 0,01 à 2,0 % en poids, rapportés au poids de tous les constituants de la matière d'enduction, à l'exception de l'eau, les radicaux $R^1$, $R^2$ et Me ayant la signification indiquée ci-dessus.

**13.** Bandelette réactive comprenant un support flexible de forme plate, sur lequel sont disposés côte à côte, dans une zone d'essai, un ou plusieurs champs d'essai qui comprennent respectivement une ou plusieurs couches fonctionnelles superposées l'une à l'autre et qui sont recouverts, le cas échéant, par un revêtement constitué d'une matière d'écartement, qui sont **caractérisées par le fait qu'**au moins une des couches fonctionnelles représente une couche fonctionnelle haute précision exempte de squelette selon l'invention selon l'une quelconque des revendications 1 à 9.

**14.** Bandelette réactive selon la revendication 13, **caractérisée en ce que** le champ d'essai comprend une feuille transparente, ainsi qu'une première couche fonctionnelle et une deuxième couche fonctionnelle appliquées sur la première citée en étant superposées l'une à l'autre, la première couche se trouvant sur la feuille transparente ayant, à l'état humide, un effet de diffusion de la lumière essentiellement inférieur à celui de la deuxième couche superposée et le côté de la feuille, qui est opposé au côté de la feuille sur lequel est appliquée la première couche, étant le côté de décèlement, et le côté de la deuxième couche qui est opposé au côté avec lequel vient s'appuyer la deuxième couche sur la première couche, étant le côté d'application de l'échantillon.

**15.** Bandelette réactive selon au moins une des revendications 13 et 14, **caractérisée en ce que** l'épaisseur de la première et de la deuxième couche fonctionnelle à l'état sec s'élève, de manière conjointe, au maximum à 0,20 mm, de préférence au maximum à 0,12 mm, de manière particulièrement préférée au maximum à 0,08 mm.

**16.** Bandelette réactive selon l'une quelconque des revendications 13 à 15, **caractérisée en ce que** la couche de support contient un trou par-dessus lequel est disposée une couche de décèlement sur laquelle sont réparties plusieurs sections réactionnelles mises les unes à côté des autres.

**17.** Utilisation d'une bandelette réactive diagnostique selon l'une quelconque des revendications 13 à 16 pour la détermination d'un analyte dans un liquide.

**18.** Procédé pour la détermination d'un analyte dans un échantillon liquide à l'aide d'une bandelette réactive diagnostique selon l'une quelconque des revendications 13 à 16, dans lequel on applique un liquide échantillon sur le côté d'application de l'échantillon et on observe la couche où les couches de décèlement en ce qui concerne la manifestation d'un signal, la manifestation d'un signal représentant une mesure de la présence, respectivement de la quantité d'un analyte dans l'échantillon liquide soumis à l'analyse.

Fig. 1

Fig. 2

Fig. 3

Fig. 4